# EUROPEAN PATENT APPLICATION

(11) **EP 4 206 192 A1**
(43) Date of publication of application: **05.07.2023**
(21) Application number: 21861516.9
(22) Date of filing: 24.08.2021
(51) Int. Cl.: C07D 311/30, A01G 7/00, A01G 22/15, A01G 22/25, A23L 33/105, A61K 8/9789, A61K 31/352, A61K 36/31, A61K 36/82, A61P 3/00, A61P 43/00, A61Q 19/00, A61Q 19/10

(54) **KAEMPFEROL AGLYCONE-CONTAINING EXTRACT**

(30) Priority: 25.08.2020 JP 2020141876; 22.12.2020 JP 2020211979
(71) Applicant: Otsuka Pharmaceutical Co., Ltd., Tokyo 101-8535 (JP)
(72) Inventor: IKEDA, Yasutaka, Osaka-shi, Osaka 540-0021 (JP); MIZOKAMI, Tsubasa, Osaka-shi, Osaka 540-0021 (JP); SERIZAWA, Ryo, Osaka-shi, Osaka 540-0021 (JP); ABIRU, Yasuhiro, Osaka-shi, Osaka 540-0021 (JP); NORO, Naoto, Osaka-shi, Osaka 540-0021 (JP)
(74) Representative: Hoffmann Eitle
(86) International application number: PCT/JP2021/030890
(87) International publication number: WO 2022/045091

(57) **Abstract**

Provided is a means useful for enjoying the physiological action of kaempferol. A plant extract contains 1 mg/g or more of a kaempferol aglycone on a dry-weight basis.

## Description

### Technical Field

Disclosed are techniques related to kaempferol aglycones, and technical matters related to techniques of improving the kaempferol production ability of plants.

### Background Art

Kaempferol is a substance represented by the following structural formula and is a type of natural flavonoid found in many edible plants such as tea, broccoli, grapefruit, cabbage, kale, beans, endive, leeks, tomatoes, strawberries, grapes, Brussel sprouts, apples, quinoa, and horseradish,

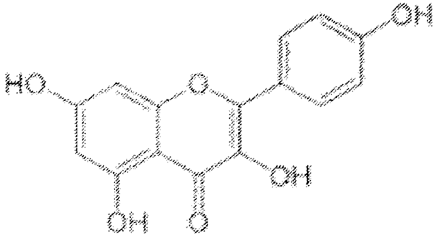

A study has recently reported that kaempferol ("KMP" below) improves oxygen consumption efficiency in vivo and has physiological action such as limiting a decrease in exercise efficiency and decreasing fatigue (PTL 1). Research also suggests that KMP may have anti-inflammatory effects, anticancer effects, and effects against various diseases, including metabolic syndrome (NPL 1) .

Kaempferol exerts useful physiological action in the form of aglycone. However, most kaempferol in plants is present in the form of glycosides. Thus, ingesting such plants or their extracts as they are provides limited beneficial physiological action of kaempferol.

Reported methods for increasing polyphenols in plants include irradiating sprouted Welsh onion (monocotyledon) with UV-B (PTL 2), growing plants with warm water (PTL 3), and decreasing the activity of flavanone-3-hydroxylase by using a molecular genetic technique (PTL 4).

### Citation List

### Patent Literature

PTL 1: WO2019/044964A
PTL 2: JP2008-86272A
PTL 3: JP2017-18056A
PTL 4: JP2003-503032A

### Non-Patent Literature

NPL 1: Exp. Ther. Med. 18: 2759-2776, 2019

### Summary of Invention

### Technical Problem

In these circumstances, an object is to provide a useful means for enjoying the physiological action of kaempferol. Another object is to improve the kaempferol production ability of plants.

### Solution to Problem

After conducting extensive investigation and research, the inventors found that specific plants contain a large amount of kaempferol, and that kaempferol glycosides in plants can be efficiently converted into kaempferol aglycones. They also found that growing plants in a reduced oxygen concentration or under reduced partial pressure of oxygen improves their kaempferol production ability. As a result of further research based on these findings, the invention, including the following subject matter, is provided.
Item A1 A plant extract comprising a kaempferol aglycone in an amount of 1 mg/g or more on a dry-weight basis.
Item A2 The plant extract according to Item A1, comprising the kaempferol aglycone in an amount of 30 mg/g or more.
Item A3 The plant extract according to Item 1A or A2, further comprising the kaempferol aglycone in an amount of 100 mg/g or more.
Item A4 The plant extract according to any one of Items A1 to A3, further comprising quercetin in an amount of 0.1 mg/g or more on a dry-weight basis.
Item A5 The plant extract according to any one of Items A1 to A4, which is an extract of a cruciferous plant.
Item A6 The plant extract according to Item A5, wherein the cruciferous plant is horseradish, kale, arugula, *takana (Brassica juncea* var. *integrifolia*), *mizuna* (*Brassica rapa* var. *nipposinica*), turnip, Japanese radish, broccoli, cabbage, a radish sprout, or bok choy.
Item A7 The plant extract according to any one of Items A1 to A4, which is an extract of a Theaceae plant.
Item A8 The plant extract according to Item A7, wherein the Theaceae plant is black tea, oolong tea, jasmine tea, green tea, *hojicha* (a type of Japanese roasted green tea), or *sencha* (a type of Japanese green tea).
Item A9 The plant extract according to any one of Items A1 to A6, which is an extract of a horseradish leaf.
Item A10 A food product, pharmaceutical product, or cosmetic product comprising the plant extract of any one of Items A1 to A9.
Item A11 A method for producing the plant extract of any one of Items A1 to A7, the method comprising
   (1) subjecting a plant material containing a kaempferol glycoside to extraction with a solvent, and
   (2) subjecting the extract obtained in step (1) to hydrolysis treatment.
Item A12 The method according to Item 9, wherein the hydrolysis treatment comprises allowing an enzyme or a microorganism to act on the extract.
Item A13 The method according to Item A11 or A12, wherein the hydrolysis treatment comprises allowing an enzyme to act on the extract, and the enzyme contains a xylanase.
Item A14 The method according to Item A13, wherein the enzyme further contains a β-glucosidase.
Item A15 The method according to Item A14, wherein the β-glucosidase is lactase.
Item B1 A method for producing a plant with an increased content of kaempferol and/or a kaempferol glycoside per unit weight (on a dry-weight basis), the method comprising growing a plant under the following condition:
   an oxygen concentration of 19 vol% or lower, or
   a partial pressure of oxygen of 193 hPa or lower.
Item B2 A method for increasing a kaempferol and/or kaempferol glycoside production ability of a plant, the method comprising growing a plant under the following condition:
   an oxygen concentration of 19 vol% or lower, or
   a partial pressure of oxygen of 193 hPa or lower.
Item B3 A method for producing a plant with an increased kaempferol and/or kaempferol glycoside production ability, the method comprising growing a plant under the following condition:
   an oxygen concentration of 19 vol% or lower, or
   a partial pressure of oxygen of 193 hPa or lower.
Item B4 The method according to any one of Items B1 to B3, wherein the condition is an oxygen concentration of 19 vol% or lower.
Item B5 The method according to any one of Items B1 to B3, wherein the condition is a partial pressure of oxygen of 50 hPa or higher and 193 hPa or lower.
Item B6 The method according to any one of Items B1 to B5, wherein the plant is a cruciferous plant.
Item B7 The method according to Item B6, wherein the cruciferous plant is horseradish, kale, arugula, *takana* (*Brassica juncea* var. *integrifolia*), *mizuna* (*Brassica rapa* var. *nipposinica*), turnip, Japanese radish, broccoli, cabbage, a radish sprout, or bok choy.
Item B8 The method according to Item B6 or B7, wherein the cruciferous plant is horseradish, Japanese radish, cabbage, or a radish sprout.
Item B9 A plant obtained by the method of any one of Items B1 to B8.
Item B10 A method for producing an extract containing kaempferol and a kaempferol glycoside, the method comprising extracting the kaempferol and kaempferol glycoside from the plant of Item 9.
Item B11 A method for producing a kaempferol-containing extract, the method comprising
   (a) extracting kaempferol and a kaempferol glycoside from the plant of Item B9, and
   (b) hydrolyzing the extract obtained in step (a).
Item B12. The method according to any one of Items B1 to B8, comprising adding L-tyrosine and/or L-phenylalanine to the plant.
Item C1 A method for increasing kaempferol production efficiency of a plant, the method comprising adding L-tyrosine and/or L-phenylalanine to a plant with a kaempferol production ability.
Item C2 A method for increasing the kaempferol content in a plant, comprising adding L-tyrosine and/or L-phenylalanine to a plant with a kaempferol production ability.
Item C3 A method for producing a plant with increased kaempferol production efficiency, the method comprising adding L-tyrosine and/or L-phenylalanine to a plant with a kaempferol production ability.
Item C4 The method according to any one of Items C1 to C3, wherein the plant is a cruciferous plant.
Item C5 The method according to Item C4, wherein the cruciferous plant is horseradish, kale, arugula, *takana (Brassica juncea* var. *integrifolia*), *mizuna* (*Brassica rapa* var. *nipposinica*), turnip, Japanese radish, broccoli, cabbage, a radish sprout, or bok choy.

### Advantageous Effects of Invention

In an embodiment, a plant extract with a high content of a kaempferol aglycone can be provided, and the physiological action by kaempferol can be efficiently enjoyed. In another embodiment, plant-derived kaempferol glycosides can be efficiently converted into kaempferol aglycones.

In another embodiment, kaempferol and/or kaempferol glycoside production ability of plants can be increased. In the present specification, kaempferol and kaempferol glycosides may be collectively referred to as "kaempferol."

### Brief Description of Drawings

Fig. 1 shows the results of the study of conditions for extraction of KMP glycosides from a plant material. Fig. 1(A) shows the test results of using water, 30% ethanol, 50% ethanol, and 70% ethanol as an extraction solvent. The solvents all had a temperature of 70°C. Fig. 1(B) shows the results of measuring the effect of solvent temperature in the use of 50% ethanol as an extraction solvent.
Fig. 2 shows the results of measuring the aglycone formation rate of KMP glycosides in the treatment of a horseradish leaf extract or quinoa extract with β-glucosidase or α-glucosidase.
Fig. 3 shows the results of measuring the aglycone formation rate of KMP glycosides in the treatment of a horseradish leaf extract with a xylanase of different concentrations.
Fig. 4 shows the results of measuring the aglycone formation rate of KMP glycosides in the treatment of an extract of horseradish leaves, black tea, Japanese radish leaves, quinoa, *takana* (*Brassica juncea* var. *integrifolia*), or kale with a xylanase.
Fig. 5 shows the results of measuring the effect of pH and temperature on conversion of KMP glycosides into aglycones in a horseradish leaf extract with a xylanase.
Fig. 6 shows the results of measuring a product obtained by treating KMP glycosides in a horseradish leaf extract with a xylanase and β-glucosidase.
Fig. 7 shows the measurement results of the amount of kaempferol and quercetin in radish sprouts grown in an oxygen concentration of 21 vol% or 17.5 vol%. "KMP" indicates kaempferol, and "Que" indicates quercetin. The vertical axis indicates the measured amount of KMP and Que (mg/g).
Fig. 8 shows the synthesis pathway around kaempferol in plants. "FLS" indicates flavonol synthase, and "F3'H" indicates flavonoid 3'-hydroxylase.
Fig. 9 shows the expression levels of the F3'H gene measured in radish sprouts and broccoli. The white bars are of cultivation in an oxygen concentration of 21 vol%, and the solid black bars are of cultivation in an oxygen concentration of 17.5 vol%.
Fig. 10 shows the expression levels of the FLS gene measured in radish sprouts and broccoli. The white bars are of cultivation in an oxygen concentration of 21 vol%, and the solid black bars are of cultivation in an oxygen concentration of 17.5 vol%.
Fig. 11 shows the measured amount of kaempferol in horseradish grown in different oxygen concentrations. The horizontal axis indicates the oxygen concentration (vol%), and the vertical axis indicates the amount of kaempferol (mg/g).
Fig. 12 shows the measured length of leaves of horseradish grown in different oxygen concentrations. The horizontal axis indicates the oxygen concentration (vol%), and the vertical axis indicates the length of leaves (cm).
Fig. 13 shows the measured length of stalks of horseradish grown in different oxygen concentrations. The horizontal axis indicates the oxygen concentration (vol%), and the vertical axis indicates the length of stalks (cm).
Fig. 14 shows the biosynthesis pathway from L-tyrosine or L-phenylalanine to kaempferol. TAL indicates tyrosine ammonia lyase, PAL indicates phenylalanine ammonia lyase, C4H indicates cinnamic acid 4-hydroxylase, 4CL indicates 4-coumaric acid CoA ligase, CHS indicates chalcone synthase, CHI indicates chalcone isomerase, F3H indicates flavanone 3-hydroxylase, FLS indicates flavonol synthase, and F3'H indicates flavonoid 3'-hydroxylase.

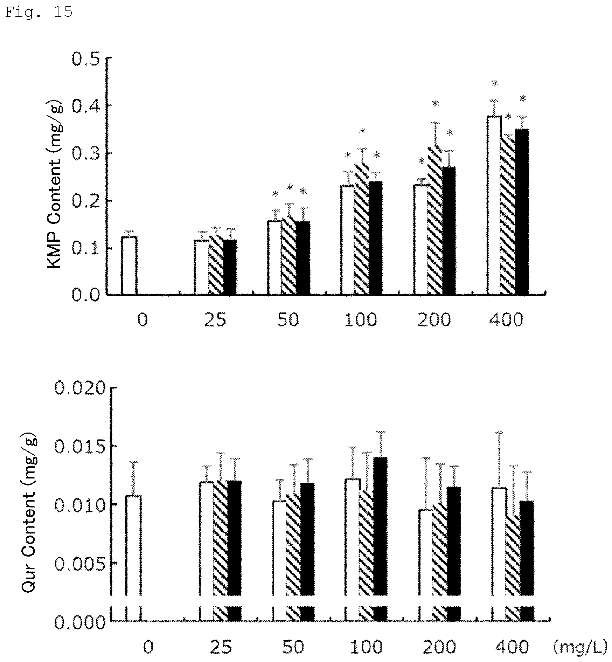

Fig. 15 shows the kaempferol (KMP) content (upper graph) and the quercetin (Qur) content in radish sprouts grown by adding an aqueous solution containing L-tyrosine and/or L-phenylalanine. The vertical axis indicates the kaempferol or quercetin content, and the horizontal axis indicate the concentration of L-tyrosine and/or L-phenylalanine in the aqueous solution. The open bars show the addition of an aqueous solution of L-phenylalanine, the shaded bars show the addition of an aqueous solution of L-tyrosine, and the black bars show the addition of an aqueous solution containing both L-phenylalanine and L-tyrosine.

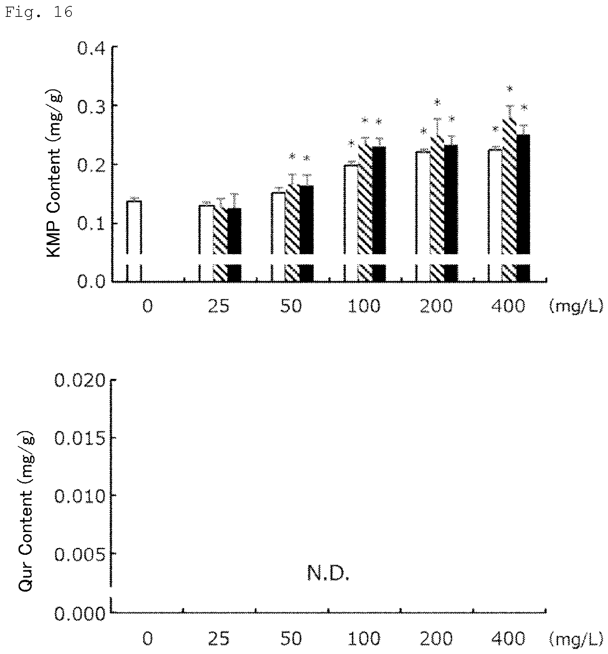

Fig. 16 shows the kaempferol (KMP) content (upper graph) and the quercetin (Qur) content in broccoli sprouts grown by adding an aqueous solution containing L-tyrosine and/or L-phenylalanine. The vertical axis indicates the kaempferol or quercetin content, and the horizontal axis indicates the concentration of L-tyrosine and/or L-phenylalanine in the aqueous solution. The open bars show the addition of an aqueous solution of L-phenylalanine, the shaded bars show the addition of an aqueous solution of L-tyrosine, and the black bars show the addition of an aqueous solution containing both L-phenylalanine and L-tyrosine.

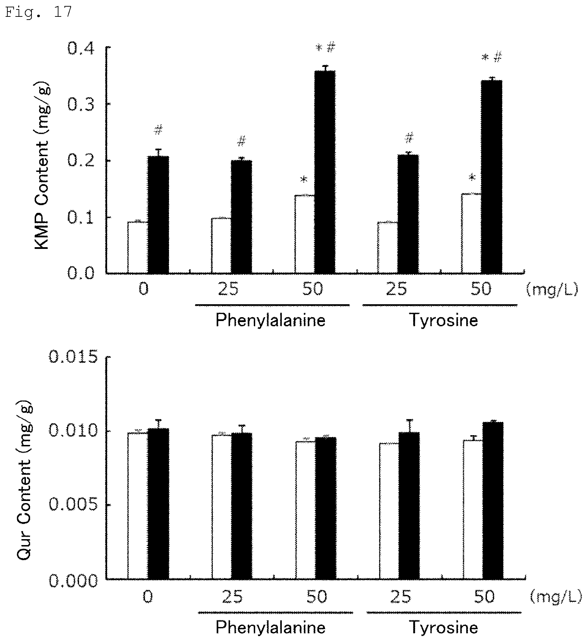

Fig. 17 shows the kaempferol (KMP) content (upper graph) and the quercetin (Qur) content in radish sprouts grown by adding an aqueous solution containing L-tyrosine or L-phenylalanine. The vertical axis indicates the kaempferol or quercetin content, and the horizontal axis indicates the concentration of L-tyrosine and/or L-phenylalanine in the aqueous solution. The open bars show the kaempferol content or the quercetin content in radish sprouts grown in an oxygen concentration of 21 vol%, and the black bars show the kaempferol content or the quercetin content in radish sprouts grown in an oxygen concentration of 17.5 vol%.

### Description of Embodiments

### A. Plant Extract and Production Method For Plant Extract

### A1. Food Product Extract

The plant extract contains a kaempferol aglycone in an amount of, for example, 1 mg/g to 500 mg/g, preferably 10 mg/g to 300 mg/g, 30 mg/g to 400 mg/g, and more preferably 100 mg/g to 200 mg/g on a dry-weight basis. The lower limit and the upper limit of the kaempferol aglycone content is not particularly limited. For example, the lower limit is 1 mg/g, 5 mg/g, 10 mg/g, 30 mg/g, 50 mg/g, or 100 mg/g. For example, the upper limit is 400 mg/g, 300 mg/g, 250 mg/g, or 200 mg/g. A preferable range of the kaempferol aglycone content can be indicated by any combination of the upper limits and lower limits above. The method for quantifying a kaempferol aglycone in a plant extract is not particularly limited, and the amount of a kaempferol aglycone can be measured according to an ordinary method. For example, the amount of a kaempferol aglycone can be measured according to the method used in the Examples described later. Kaempferol is preferably measured at a pH of 2 to 7.

Ingestion of a plant extract with a high content of a kaempferol aglycone leads to improved oxygen consumption efficiency in vivo, and provides effects such as limiting decreases in exercise efficiency, alleviation of fatigue, and limiting decreases in dynamic visual acuity.

The plant extract preferably contains quercetin (in aglycone form) in addition to the kaempferol aglycone. The quercetin content in the plant extract on a dry-weight basis is, for example, 0.1 mg/g to 100 mg/g, preferably 1 mg/g to 30 mg/g, and more preferably 5 mg/g to 10 mg/g. The lower limit and the upper limit are not particular limited. For example, the lower limit is 0.1 mg/g, 0.5 mg/g, 1 mg/g, 2.5 mg/g, or 5 mg/g. For example, the upper limit is 100 mg/g, 50 mg/g, 30 mg/g, 20 mg/g, or 10 mg/g. A preferable range of the quercetin content can be indicated by any combination of the upper limits and the lower limits above. The method for quantifying quercetin in a plant extract is not particularly limited, and the amount of quercetin can be measured according to an ordinary method. For example, the amount of quercetin can be measured according to the method used in the Examples described later.

In an embodiment, the content of quercetin in aglycone form in the plant extract is, on a dry-weight basis, preferably 1/2000 or more, 1/1000 or more, 1/500 or more, 1/100 or more, or 1/50 or more, and preferably 1/10 or less, 1/20 or less, 1/50 or less, 1/100 or less, or 1/500 or less of the content of kaempferol in aglycone form. These upper limits and lower limits can be combined in any way.

The plant extract, by containing quercetin, can effectively provide physiological action such as antioxidant effects, anti-inflammatory effects, anti-arteriosclerosis effects, cerebrovascular disease prevention, antitumor effects, antihypertensive effects, and/or vasorelaxant effects.

The plant extract preferably contains ferulic acid in addition to a kaempferol aglycone. The ferulic acid content in the plant extract is, for example, preferably 0.1 mg/g or more, 0.2 mg/g or more, 0.3 mg/g or more, 0.4 mg/g or more, 0.5 mg/g or more, 0.6 mg/g or more, 0.7 mg/g or more, 0.8 mg/g or more, 0.9 mg/g or more, 1 mg/g or more, 1.1 mg/g or more, 1.2 mg/g or more, 1.3 mg/g or more, 1.4 mg/g or more, 1.5 mg/g or more, 1.6 mg/g or more, 1.7 mg/g or more, 1.8 mg/g or more, 1.9 mg/g or more, or 2 mg/g or more, on a dry-weight basis. The upper limit of the ferulic acid content is not particularly limited, and can be set to, for example, 10 mg/g, 9 mg/g, 8 mg/g, 7 mg/g, 6 mg/g, 5 mg/g, 4 mg/g, 3 mg/g, or 2 mg/g. The method for quantifying ferulic acid in the plant extract is not particularly limited, and the amount of ferulic acid can be measured according to an ordinary method.

The plant extract, by containing ferulic acid, can effectively provide physiological action, such as antioxidant effects and/or antitumor effects.

In an embodiment, the plant extract is preferably an extract of a cruciferous plant or a Theaceae plant. Examples of cruciferous plants include horseradish, kale, arugula, *takana* (*Brassica juncea* var. *integrifolia*), *mizuna* (*Brassica rapa* var. *nipposinica*), turnip, Japanese radish, broccoli, cabbage, radish sprouts, and bok choy. Examples of Theaceae plants include black tea, oolong tea, jasmine tea, green tea, *hojicha* (a type of Japanese roasted green tea), and *sencha* (a type of Japanese green tea). In an embodiment, the plant extract is preferably an extract of plants described below in section "B. Method for Increasing Kaempferol Production Ability and Plant with Increased Kaempferol Production Ability."

In an embodiment, the plant extract may be of a plant other than cruciferous or Theaceae plants. Examples of such plants include saffron, *Gymnema sylvestre,* onion skin, garlic chives, parsley, and millet such as quinoa, canary beans, and lentils.

The plant extract can be an extract of any part of a plant. For example, the plant extract can be an extract of a whole plant, leaves, stems, roots, or flowers, or any combination of these. For example, if a cruciferous plant or Theaceae plant is used as a raw material, it is preferable to use the leaves of the plant. For plants other than cruciferous or Theaceae plants listed above, it is also preferable to use leaves of the plants, except for onions.

In an embodiment, the plant extract is preferably an extract of horseradish, and preferably an extract of leaves of horseradish. This is because horseradish leaves contain a large amount of kaempferol glycosides.

The plant extract is preferably obtained by not only subjecting a plant material to extraction but also further subjecting glycosides to treatment for converting the glycosides into aglycones. Although the plant extract described above can be obtained according to any method, the plant extract is preferably obtained by the method for producing a plant extract described later.

The plant extract described above can be processed into any product; for example, food products, pharmaceutical products, or cosmetic products containing the plant extract can be provided. The plant extract for use for such purposes may be an extract treated with an enzyme as is, or may be an extract further purified. The plant extract can also be optionally subjected to drying treatment to form the extract into a dry solid. To improve the storage stability of the plant extract, it is preferable to form the plant extract into a solid by drying treatment (including lyophilization). The dried plant extract may also be subjected to powdering treatment to form the extract into powder, if necessary.

The plant extract for use as a food material can be prepared in the form of, for example, granules, fine granules, capsules, tablets, powders, beverages (e.g., soft drinks, carbonated drinks, nutritional drinks, powdered drinks, fruit drinks, dairy drinks, and jelly drinks), dairy products, confectionery (e.g., cookies, biscuits, chocolate confections, chips, cake, gum, candies, gummy candies, buns, adzuki-bean jelly, pudding, jelly, yogurt, ice cream, and sherbet), butter, other solid food products, or semisolid food products. Food products containing the plant extract can be, for example, foods for specified health use, foods with functional claims, diet supplementary food, functional food products, foods for sick people, as well as general food products.

The proportion of the plant extract in a food product can be set as appropriate according to, for example, the type and intended use of the food product, the kaempferol content, the age and gender of the target subject for intake of the food product, and expected effects. For example, a food product preferably contains the plant extract on a dry-weight basis in an amount of 2 mg or more, 10 mg or more, 50 mg or more, 100 mg or more, 500 mg or more, or 1 g or more, and preferably 50 g or less, 30 g or less, 10 g or less, 5 g or less, or 2 g or less, per 100 g of the food product. These lower limits and upper limits of the content of the plant extract can be combined in any way. For example, a food product may contain the plant extract in an amount of 2 mg or more and 50 g or less, 10 mg or more and 30 g or less, or 50 mg or more and 10 g or less, per 100 g of the food product on a dry-weight basis. Alternatively, for example, the kaempferol aglycone content on a dry-weight basis is preferably 0.1 mg or more, 0.5 mg or more, 1 mg or more, or 2 mg or more, and preferably 1000 mg or less, 100 mg or less, 20 mg or less, 10 mg or less, or 5 mg or less, per 100 g of a food product. These lower limits and upper limits can be combined in any way. For example, a food product may contain a kaempferol aglycone on a dry-weight basis in an amount of 0.1 mg or more and 1000 mg or less, 0.5 mg or more and 100 mg or less, or 1 mg or more and 20 mg or less, per 100 g of the food product.

The daily intake of a food product containing the plant extract varies according to the age and body weight of the subject who ingests the food product, and the purpose of the intake. For example, the intake of a food product can be adjusted such that the daily intake of the plant extract on a dry-weight basis is 2 mg or more, 10 mg or more, or 50 mg or more, and 200 mg or less, 150 mg or less, or 100 mg or less. Alternatively, the intake of kaempferol aglycones is preferably 0.1 mg or more, 1 mg or more, 2 mg or more, 3 mg or more, or 5 mg or more, and preferably 100 mg or less, 50 mg or less, 40 mg or less, 20 mg or less, or 12 mg or less. These lower limits and upper limits can be combined in any way. For example, the daily intake of a food product containing the plant extract can be adjusted such that the daily intake of kaempferol aglycones is 0.1 mg or more and 100 mg or less, 1 mg or more and 50 mg or less, or 2 mg or more and 40 mg or less.

The plant extract for use as a pharmaceutical material can be prepared into a pharmaceutical formulation in the form of, for example, tablets, pills, powdered drugs, fluid medicines, suspensions, emulsions, granules, capsules, aerosol agents, patches, injectable drugs, or suppositories. The dose of such a pharmaceutical product varies according to the age, body weight, symptoms, frequency of administration, and purpose of administration for the subject of administration and cannot be uniformly determined. For example, the daily dose of the plant extract for an adult can be 0.1 mg/kg to 10 g/kg (body weight), and preferably 1 mg/kg to 5 g/kg (body weight) on a dry-weight basis.

The plant extract for use as a cosmetic material can be prepared in various desired forms, such as of paste, mousse, gel, liquid, emulsion, suspension, cream, ointment, and sheet. These cosmetics are usable as basic cosmetics such as emulsions, creams, lotions, oils, and packs; cleaners such as facial washes, cleansers, and body washes; wipes; and sanitizers.

The proportion of the plant extract in a cosmetic product can be determined as appropriate according to, for example, the type of cosmetic, kaempferol content, and intended use. For example, the proportion of the plant extract is 0.01 g to 95 g, preferably 0.1 g to 50 g, and more preferably 1 g to 5 g on a dry-weight basis per 100 g of the cosmetic.

### A2. Method for Producing Food Product Extract

Although the method for producing the plant extract above is not particularly limited, the method preferably includes subjecting a plant material containing kaempferol glycosides to extraction with a solvent and subjecting the obtained extract to hydrolysis treatment (treatment for converting glycosides into aglycones).

The plant material may be raw, dried, or roughly dried. From the viewpoint of efficient extraction of kaempferol or glycosides of kaempferol, the plant material is preferably ground according to an ordinary method before use. In an embodiment, the plant material for use is preferably those described below in section "B. Method for Increasing Kaempferol Production Ability and Plant with Increased Kaempferol Production Ability."

The extraction method is not particularly limited, and extraction can be performed according to a method commonly used in the field of pharmaceutical formulations or food engineering. The extraction method is, for example, extraction performed by using water, or at least one organic solvent selected from the group consisting of ethanol, methanol, butanol, ether, ethyl acetate, and chloroform, or a mixed solvent of any of these organic solvents and water. In an embodiment, extraction is preferably performed at 30 to 70°C by using an aqueous solution of 30 to 70% ethanol. The pH during extraction is preferably 3 to 7.

The plant extract obtained by extraction with water or an organic solvent may be subjected to purification treatment such as column purification or solid-liquid separation before hydrolysis treatment.

The means for hydrolysis treatment (conversion of glycosides into aglycones) is not particularly limited. Hydrolysis treatment can be performed by using enzymes, microorganisms, acids, and/or bases.

When conversion to aglycones is performed by using an enzyme, the enzyme for use is preferably selected according to the type of carbohydrates that constitute the kaempferol glycosides and glycoside structure. The carbohydrates that constitute kaempferol glycosides possibly contained in the plant extract include glucose, galactose, rhamnose, xylose, and combinations of these (e.g., sophorose (glucose + glucose), rutinose (rhamnose + glucose), and neohesperidose (rhamnose + glucose)). These carbohydrates are O-glycosidically bound to the 3-, 5-, and/or 7-positions of kaempferol to form glycosides.

The structure of kaempferol glycosides varies according to the species of the plant. The relationship between specific plant species and the type of carbohydrates that constitute glycosides is, for example, as follows:
Horseradish leaf: galactose + xylose
Saffron: glucose;
Tea leaf: glucose, galactose + rhamnose + glucose, xylose + rhamnose + glucose, galactose + glucose, glucose + rutinose (rhamnose + glucose);
Kale: glucose + glucose *Takana* (*Brassica juncea* var. *integrifolia*): glucose + glucose + glucose
Arugula: glucose

Examples of enzymes suitable for converting these kaempferol glycosides into aglycones include xylanase, lactase, glucosidase, arabinosidase, rhamnosidase, xylosidase, cellulase, hesperidinase, naringinase, glucuronidase, pectinase, galactosidase, amyloglucosidase, and amylase. These can be used singly, or in a combination of two or more. For example, kaempferol glycosides contained in an extract of horseradish leaves are converted into aglycones preferably by using xylanase, more preferably by using a combination of xylanase and β-glucosidase, and still more preferably by using a combination of xylanase and lactase. Kaempferol glycosides contained in an extract of kale, *takana* (*Brassica juncea* var. *integrifolia*), Japanese radish leaves, black tea, or quinoa are converted into aglycones preferably by using β-glucosidase.

In an embodiment, when xylanase and β-glucosidase (e.g., lactase) are used in combination in conversion to aglycones, the amount of xylanase and β-glucosidase is not particularly limited. For example, from the viewpoint of efficient conversion to aglycones, the weight ratio of xylanase to β-glucosidase (xylanase:β-glucosidase) is within the range of 10:0.5 to 10:10, and preferably 10:2 to 10:4.

In conversion of kaempferol glycosides into aglycones by using a microorganism, microorganisms producing the enzymes described above are preferably used. Examples of such microorganisms include microorganisms of the genus *Lactobacillus*, the genus *Lactococcus*, the genus *Aspergillus*, the genus *Bacillus*, the genus *Penicillium*, the genus *Rhizopus*, the genus *Rhizomucor*, the genus *Talaromyces*, the genus *Bifidobacterium*, the genus *Mortierella*, the genus *Cryptococcus*, or the genus *Microbacterium.* These microorganisms can be used singly, or in a combination of two or more. For example, kaempferol glycosides contained in an extract of horseradish leaves can be converted into kaempferol aglycones by using microorganisms of the genus *Lactobacillus*, preferably at least one member selected from the group consisting of *L. pentosus*, *L. plantarum*, *L. casei*, *L. sakei*, *L. kefirgranum, L. brevis*, and *L. tucceti*; and more preferably at least one member selected from the group consisting of *L. pentosus*, *L. plantarum*, *L. casei*, and *L. tucceti.*

It is preferable to convert glycosides into aglycones by adding a microorganism to a plant extract and, for example, culturing the microorganism under conditions suitable for growth. Suitable conditions for growth can be set for each kind of microorganism, and can be designed as appropriate, for example, 20 to 40°C and a pH of 4 to 7.

The acid usable in conversion of kaempferol glycosides into aglycones is, for example, one or more acids selected from the group consisting of hydrochloric acid, sulfuric acid, and nitric acid, or a mixed solvent of any of these acids with one or more alcohols selected from the group consisting of ethanol, methanol, and butanol. The concentration of the acid is, for example, but not limited to, 0.1 to 2 N. The alcohol content in the mixed solvent is, for example, but not limited to, 50 to 70%. The reaction temperature is, for example, 50 to 100°C, and the reaction time can be designed within the range of, for example, 0.5 to 24 hours.

The base usable in conversion of kaempferol glycosides into aglycones is, for example, one or more bases selected from the group consisting of sodium hydroxide and potassium hydroxide, or a mixed solvent of any of these bases with one alcohol selected from the group consisting of ethanol, methanol, and butanol. The concentration of the base is, for example, but not limited to, 0.1 to 0.5 N. The alcohol content in the mixed solvent is, for example, but not limited to, 50 to 70 v/v%. The reaction temperature is, for example, 50 to 100°C, and the reaction time can be designed within the range of, for example, 0.5 to 24 hours.

### B. Method for Increasing Kaempferol Production Ability and Plant with Increased Kaempferol Production Ability

The method for producing a plant with an increased content of kaempferol and/or a kaempferol glycoside, the method for increasing a kaempferol and/or kaempferol glycoside production ability of a plant, and the method for producing a plant with an increased kaempferol and/or kaempferol glycoside production ability preferably include growing a plant in an oxygen concentration lower than about 20.9 vol%, which is the oxygen concentration in the natural environment, or under a partial pressure of oxygen lower than about 213 hPa. The oxygen concentration in the environment in which a plant is grown can be measured with a commercially available oxygen concentration meter. The oxygen concentration can also be measured with a device that controls the oxygen concentration or partial pressure of oxygen in the air, described below.

The oxygen concentration lower than about 20.9 vol% is preferably about 20 vol% or lower, about 19.5 vol% or lower, about 19 vol% or lower, about 18.5 vol% or lower, about 18 vol% or lower, about 17.5 vol% or lower, about 17 vol% or lower, about 16.5 vol% or lower, or about 16 vol% or lower. The lower limit of the oxygen concentration can be any at which plants can grow, and can be set to, for example, 10 vol% or higher, 11 vol% or higher, 12 vol% or higher, 13 vol% or higher, 14 vol% or higher, 15 vol% or higher, or 16 vol% or higher. These upper limits and lower limits can be combined in any way.

The partial pressure of oxygen lower than about 213 hPa is preferably, for example, 203 hPa or lower, 198 hPa or lower, 193 hPa or lower, 188 hPa or lower, 183 hPa or lower, 178 hPa or lower, 173 hPa or lower, 168 hPa or lower, or 163 hPa or lower. The lower limit of the partial pressure of oxygen can be any at which plants can grow, and can be set to, for example, 50 hPa or higher, 60 hPa or higher, 70 hPa or higher, 80 hPa or higher, 90 hPa or higher, 102 hPa or higher, 112 hPa or higher, 122 hPa or higher, 132 hPa or higher, 142 hPa or higher, 152 hPa or higher, or 163 hPa or higher.

The method for controlling the oxygen concentration or partial pressure of oxygen at which a plant is grown as described above is not limited. For example, a plant can be grown in an environment, in a facility, or in a device in which the plant can be grown while the oxygen concentration or partial pressure of oxygen is controlled so as to fall within a predetermined range. For example, agricultural greenhouses, plant factories, and other containers or covers that can grow plants are usable. As long as the oxygen concentration or partial pressure of oxygen in the air with which the plant is in contact is within the ranges above, the oxygen concentration or the partial pressure of oxygen of the entire environment in which the plant is grown does not have to be completely within the above ranges.

The means for controlling the oxygen concentration or partial pressure of oxygen can be any means. Examples include selectively removing oxygen from the environment in which the plant is grown, supplying gases other than oxygen (e.g., nitrogen or carbon dioxide) to the environment in which the plant is grown, and supplying air with a pre-controlled oxygen concentration or partial pressure of oxygen to the environment in which the plant is grown. Because there are various known devices for supplying air with a controlled oxygen concentration, such devices can be selected for use as appropriate.

In an embodiment, when growing a plant in an oxygen concentration lower than about 20.9 vol% or under a partial pressure of oxygen lower than about 213 hPa, it is preferable to grow the plant while monitoring the oxygen concentration or partial pressure of oxygen under which the plant is present. The frequency of monitoring can be designed as appropriate, for example, within the range of 3 times/day to 1 time/week, specifically 3 times/day, 2 times/day, 1 time/day, 1 time/2 days, 1 time/3 days, 1 time/4 days, 1 time/5 days, 1 time/6 days, or 1 time/7 days.

The conditions for growing a plant other than the oxygen concentration and partial pressure of oxygen can be designed as appropriate within the range of conditions under which the plant can grow, preferably conditions suitable for plant growth. For example, the light source may be natural light (sunlight) or an artificial light source, and irradiation with light such as ultraviolet light (e.g., UV-A and UV-B) may or may not be performed. The cultivation method can be either hydroponics (solid media cultivation, water cultivation, or aeroponics) or conventional soil cultivation. If temperature conditions are controlled, the conditions can be appropriately designed, such as a temperature within the range of 10°C or higher and 35°C or lower, or 15°C or higher and 30°C or lower. The location where plants are grown is not limited. In an embodiment, for example, the location 0 m or higher and 1000 m or lower, 0 m or higher and 800 m or lower, or 0 m or higher and 600 m or lower above sea level can be selected as appropriate. The concentration of gases other than oxygen (e.g., carbon dioxide) may or may not be controlled (decreased or increased) according to the plant species and purposes.

In an embodiment, it is preferable to grow a plant by adding L-tyrosine and/or L-phenylalanine. Feeding a plant with L-tyrosine and/or L-phenylalanine promotes the biosynthesis of kaempferol by the plant. The method of adding L-tyrosine and/or L-phenylalanine to a plant is not particularly limited and can be any method. For example, a solution of L-tyrosine and/or L-phenylalanine in a solvent such as water can be given to a plant or to plant-growing soil, or L-tyrosine and/or L-phenylalanine can be added directly to plant-growing soil. The amount of L-tyrosine and/or L-phenylalanine to be given is not particularly limited and can be set appropriately, taking into account, for example, the species, size, growing environment, and growth conditions of the plant. The solution of L-tyrosine and/or L-phenylalanine in water to be added to a plant may have a concentration of L-tyrosine and/or L-phenylalanine of, for example, 30 mg/L or higher, 35 mg/L or higher, 40 mg/L or higher, 45 mg/L or higher, 50 mg/L, 60 mg/L or higher, 70 mg/L or higher, 80 mg/L or higher, 90 mg/L or higher, 100 mg/L or higher, 150 mg/L or higher, 200 mg/L or higher, 300 mg/L or higher, 400 mg/L or higher, or 500 mg/L or higher. The upper limit of the L-tyrosine and/or L-phenylalanine concentration in the aqueous solution is not limited, and can be, for example, 2000 mg/L or lower, 1500 mg/L or lower, 1000 mg/L or lower, 500 mg/L or lower, 400 mg/L or lower, 300 mg/L or lower, 200mg/or less, or 100 mg/L or lower. These upper limits and lower limits of the concentration can be combined in any way. L-tyrosine and/or L-phenylalanine may be added to a plant only once or continuously over a period of time. The period of time is, for example, 1 day or more, 2 days or more, 3 days or more, 4 days or more, 5 days or more, 6 days or more, 1 week or more, 2 weeks or more, 3 weeks or more, 4 weeks or more, or one month or more, and one year or less, 6 months or less, 5 months or less, 4 months or less, 3 months or less, 2 months or less, one month or less, 4 weeks or less, 3 weeks or less, 2 weeks or less, or 1 week or less. These lower limits and upper limits can be combined in any way.

The period of time for growing a plant in an oxygen concentration lower than about 20.9 vol% or under a partial pressure of oxygen lower than about 213 hPa is not limited and can be designed as appropriate, such as one day to several months, according the plant species and purposes. The time at which a plant is grown in an oxygen concentration lower than about 20.9 vol% or under a partial pressure of oxygen lower than about 213 hPa in the growing process of the plant is not limited and can be, for example, at least one selected from the group consisting of sowing time, germination time, growing period, pre-harvest period, post-harvest period, and a combination of these.
In an embodiment, it is preferable to grow a plant in an oxygen concentration lower than about 20.9 vol% or under a partial pressure of oxygen lower than about 213 hPa over the entire period from sowing to harvest.

Although the plant can be of any species, the plant preferably has a kaempferol production ability. The plant with a kaempferol production ability may be a plant that inherently has a kaempferol production ability, or a plant that has artificially acquired a kaempferol production ability by a genetic engineering procedure etc. In an embodiment, the plant is preferably a cruciferous plant or Theaceae plant, and preferably a cruciferous plant.

Examples of cruciferous plants include horseradish, kale, arugula, *takana* (*Brassica juncea* var. *integrifolia*), *mizuna* (*Brassica rapa* var. *nipposinica*), turnip, Japanese radish, broccoli, cabbage, radish sprouts, and bok choy. In an embodiment, cruciferous plants are preferably horseradish, Japanese radish, cabbage, and radish sprouts. Examples of Theaceae plants include black tea, oolong tea, jasmine tea, green tea, *hojicha* (a type of Japanese roasted green tea), and *sencha* (a type of Japanese green tea) .

Plants with a kaempferol production ability other than cruciferous and Theaceae plants include saffron, *Gymnema sylvestre*, onion skin, garlic chives, parsley, and millet such as quinoa, canary beans, and lentils.

The plant grown by the method described above has a higher kaempferol or kaempferol glycoside production ability and/or a higher kaempferol or kaempferol glycoside content than plants grown in an oxygen concentration of about 20.9 vol%, which is the oxygen concentration in the natural environment, or under a partial pressure of oxygen of about 213 hPa. The plant grown by the method described above is preferably not accompanied by growth inhibition (e.g., hypocotyl shrinkage and leaf area reduction). The amount of kaempferol and/or kaempferol glycosides in a plant can be measured according to the method used in the Examples, described later.

In an embodiment, a method for producing an extract containing kaempferol and kaempferol glycosides including extracting kaempferol and kaempferol glycosides from a plant grown according to the method described above is provided. An extract with a high content of kaempferol and/or kaempferol glycosides can be efficiently obtained by using the plant grown according to the method described above as a raw material.

The extraction method is not particularly limited, and extraction can be performed according to a method commonly used in the field of pharmaceutical formulations or food engineering. The extraction method is, for example, extraction performed by using water, or at least one organic solvent selected from the group consisting of ethanol, methanol, butanol, ether, ethyl acetate, and chloroform, or a mixed solvent of any of these organic solvents and water. In an embodiment, extraction is preferably performed at 30 to 70°C by using an aqueous solution of 30 to 70% ethanol. The pH during extraction is preferably 3 to 7.

In an embodiment, a method for producing a kaempferol-containing extract including hydrolyzing kaempferol glycosides extracted from a plant grown according to the method described above is provided. An extract with a high content of kaempferol can be efficiently obtained by using a plant grown according to the method described above as a raw material. The means for performing hydrolysis (conversion of glycosides into aglycones) is not particularly limited, and hydrolysis can be performed by using enzymes, microorganisms, acids, and/or bases. For example, the kaempferol-containing extract can be obtained according to the extraction method described in section A above. The kaempferol-containing extract has the applications and is usable for the purposes, and in the forms described in section A above.

C. The method for increasing the kaempferol production efficiency of a plant, the method for increasing the kaempferol content in a plant, and the method for producing a plant with an increased kaempferol production efficiency preferably include adding L-tyrosine and/or L-phenylalanine to the plant. The kind of the plant, the technique of adding L-tyrosine and/or L-phenylalanine to the plant, etc. are as described in section B above.

### Examples

The following describes the present invention in more detail with reference to Examples. However, the present invention is not limited these Examples.

### A1. Measurement of KMP Amount in Materials

The total KMP amount in each plant material was measured according to the following procedure. After each material was ground with a mill, 0.8 g of the ground material was taken, and 40 mL of 70% ethanol was added, followed by suspending it with a Polytron homogenizer at room temperature at 20,000 rpm for 1 minute. 1 mL of the suspension was dispensed and weighed into a glass test tube. 1 mL of 2N hydrochloric acid was added, and the mixture was mixed with a vortex and heated at 100°C for 20 minutes with a heat block. After heating was completed, the mixture was cooled with ice for at least 5 minutes. After cooling, 5 mL of hexane was added, and the mixture was shaken horizontally 20 times. After centrifugation at 3,000 rpm for 5 minutes at 4°C with a cooled centrifuge, the upper layer was discarded. 5 mL of ethyl acetate was added, and the mixture was shaken horizontally with a shaker at room temperature at 250 rpm for 10 minutes. After centrifugation with a large cooled centrifuge at 4°C at 3,000 rpm for 5 minutes, the upper layer was collected in a glass test tube for drying. 5 mL of ethyl acetate was added to the lower layer again, and the mixture was shaken horizontally with a shaker at room temperature at 250 rpm for 10 minutes. After centrifugation at 4°C at 3,000 rpm for 5 minutes, the upper layer was collected in a glass test tube for drying. The collected upper layer was dried with nitrogen at 60°C with a nitrogen spray dryer. After completion of drying, 1 mL of an aqueous solution of 70% ethanol was added, and the mixture was treated with an ultrasonic generator for 1 minute to dissolve it with a vortex. After being passed through a 0.45-µm filter, the solution was subjected to HPLC for analyzing KMP aglycones.

Due to the presence of few foreign substances, the raw material suspension, extract, and aglycone liquid of horseradish leaves were measured for (1) the total amount of KMP or (2) the amount of KMP aglycones according to the following simple method.
(1) Measurement of the total amount of KMP (acid hydrolysis): 1 mL of a sample solution was dispensed and weighed into a glass test tube. 1 mL of 2N hydrochloric acid was added, and the mixture was mixed with a vortex and heated at 100°C for 20 minutes with a heat block. After heating was completed, the mixture was cooled with ice for at least 5 minutes. After cooling, 960 µL of a 2N sodium hydroxide solution was added and mixed to adjust the pH to 4 to 7. The neutralized solution was all transferred to a 10-mL volumetric flask with a Pasteur pipette, and the volume was adjusted to 10 mL with a 70% ethanol solution, followed by mixing. After being passed through a 0.45-µm filter, the solution was subjected to HPLC for analyzing KMP aglycones, and the total amount of KMP was determined.
(2) Measurement of the amount of KMP aglycones: 1 mL of a sample solution was dispensed and weighed into a 10-mL volumetric flask, and the volume was adjusted to 10 mL with a 70% ethanol solution, followed by mixing. After being passed through a 0.45-µm filter, KMP aglycone analysis was performed by HPLC, and the amount of KMP aglycones was determined.

As shown in Table 1 below, a high total content of KMP was found in cruciferous plants such as horseradish leaves, kale, arugula, *takana* (*Brassica juncea* var. *integrifolia*), and *Karashina (Vegetable Mustard);* Theaceae plants such as black tea leaves, oolong tea, jasmine tea, green tea, and *hojicha*; and millet such as quinoa, canary beans, and lentils.

**Table 1**

| No. | Material | KMP Content (mg/g) |
|---|---|---|
| 1 | Horseradish Leaves | 35.5 |
| 2 | Saffron Powder | 4.54 |
| 3 | Black Tea Leaves | 2.57 |
| 4 | Kale | 1.74 |
| 5 | Propolis | 1.41 |
| 6 | Arugula | 1.2 |
| 7 | Persimmon Leaf Tea | 1.15 |
| 8 | Green Tea | 1.09 |
| 9 | Quinoa | 0.66 |
| 10 | Broccoli | 0.65 |
| 11 | *Takana* | 0.59 |
| 12 | *Urui* (Hostas) | 0.59 |
| 13 | Canary Beans | 0.35 |
| 14 | *Karashina* (Vegetable Mustard) | 0.29 |
| 15 | Lentils | 0.28 |
| 16 | Garlic Chives | 0.21 |
| 17 | Parsley | 0.2 |
| 18 | Split Scallion | 0.14 |
| 19 | Bok Choy | 0.13 |
| 20 | Ginkgo | 0.12 |

### A2. Study of Extraction Conditions for KMP Glycoside

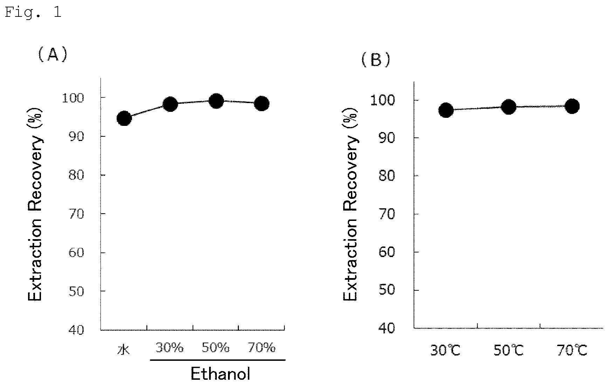

The conditions of extraction solvents and extraction temperature in the extraction of KMP glycosides from plants were studied. 75 g of water or an aqueous solution of 30 to 70% ethanol (extraction solvent) was added to 1.5 g of horseradish leaves, and an extraction operation was performed at 70°C two times. Separately, 75 g of an aqueous solution of 50% ethanol (extraction solvent) was added to 1.5 g of horseradish leaves, and an extraction operation was performed at 30 to 70°C two times. The entire amount of the obtained extract was weighed, and then the amount of KMP aglycones was measured as described in section "(2) Measurement of the amount of KMP aglycones" above. The extraction recovery was determined based on the sum of the amount of KMP in the extraction residue and the amount of KMP in the extraction solution taken as 100%. Fig. 1 shows the extraction recovery of KMP under different conditions. As shown in Fig. 1, the study confirmed that KMP glycosides of horseradish leaves were able to be recovered with hot-water extraction or extraction with 30 to 70% ethanol, and that the favorable extraction temperature was 30 to 70°C.

### A3. Conversion of KMP Glycosides into KMP Aglycones

### A3-1. Aglycone Formation Test 1 (conversion of glycosides into aglycones by β-glucosidase)

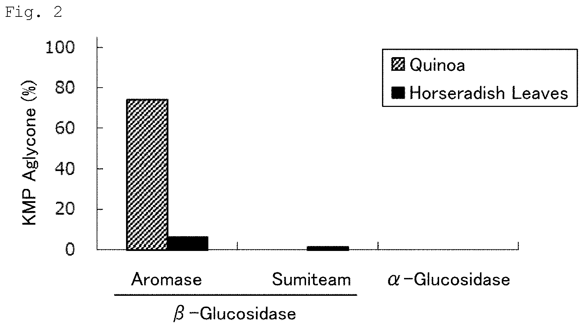

A hot-water extract of horseradish leaves or quinoa was prepared and adjusted to give 50 ug/mL of KMP aglycones. β-glucosidase (Aromase H2, Amano Enzyme Inc.), β-glucosidase (Sumiteam BGA, *Shin Nihon Kagaku Kogyo K.K.*), or α-glucosidase (Amano Enzyme Inc.) was added to the prepared reaction solution so as to give a final concentration of 500 ug/mL and allowed to react at 60°C for 60 minutes, followed by measuring the KMP aglycone concentration by HPLC. Fig. 2 shows the aglycone formation rate based on the KMP aglycone concentration during acid hydrolysis taken as 100%. The conversion of glycosides into aglycones of the quinoa extract was not performed in the tests using an enzyme other than β-glucosidase (Aromase H2).

As shown in Fig. 2, when β-glucosidase (Aromase H2) was used, about 75% of the KMP glycosides in the quinoa extract were converted into aglycones. However, the KMP glycosides in the horseradish leaf extract were converted into aglycones by only about 2 to 5%. Similar results were also confirmed in the test using another type of β-glucosidase (Sumiteam BGA). The conversion of glycosides into aglycones was not confirmed with α-glucosidase.

### A3-2. Aglycone Formation Test 2 (conversion of glycosides into aglycones by xylanase)

A hot-water extract of horseradish leaves was prepared and adjusted to give 50 ug/mL of KMP aglycones with a pH of 5.0. Hemicellulase Amano 90 was added to the prepared reaction solution to give a final concentration of 250, 500, 1000, or 1500 ug/mL and allowed to react at 50°C for 5 hours, followed by measuring the KMP aglycone concentration by HPLC. The aglycone formation rate was indicated based on the KMP aglycone concentration during acid hydrolysis taken as 100%.

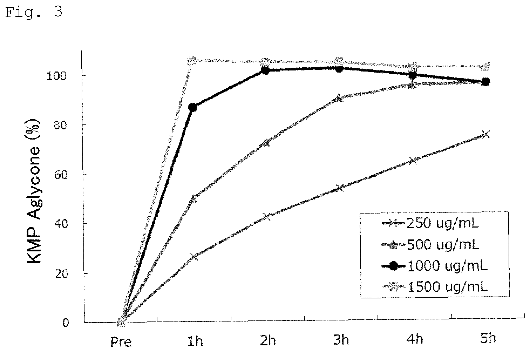

As shown in Fig. 3, in the treatment of a hot-water extract of horseradish leaves with a xylanase (Hemicellulase Amano 90, Amano Enzyme Inc.), whereas the enzyme in an amount of 250 ug/mL took 5 hours for reaction to achieve 70% conversion of glycosides into aglycones, the enzyme in an amount of 500 ug/mL was able to achieve more than 90% conversion of glycosides into aglycones in 4 hours. The enzyme in an amount of 1000 ug/mL was able to achieve 100% conversion of glycosides into aglycones in 2 hours, and the enzyme in an amount of 1500 ug/mL was able to achieve more than 100% conversion of glycosides into aglycones in 1 hour. These results indicate that xylanases are effective in converting the KMP glycosides in a horseradish leaf extract into aglycones.

### A3-3. Conversion of KMP Glycosides in Various Plant Extracts Into Aglycones By Xylanase

Hot-water extracts of kale, *takana (Brassica juncea* var. *integrifolia*), quinoa, Japanese radish leaves, black tea leaves, and horseradish leaves were prepared and adjusted to give 25 ug/mL of KMP aglycones with a pH of 5.0. A xylanase (Hemicellulase Amano 90) was added to each of the prepared reaction solution to give a final concentration of 1250 ug/mL and allowed to react at 50°C for 6 hours, followed by measuring the KMP aglycone concentration by HPLC. The aglycone formation rate was indicated based on the KMP aglycone concentration during acid hydrolysis taken as 100%.

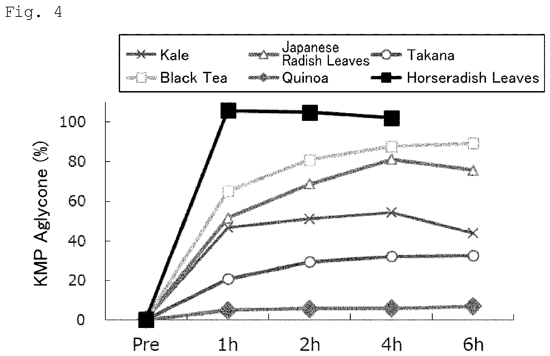

As shown in Fig. 4, even though the quinoa extract was treated with the xylanase, glycosides were hardly converted into aglycones. The extracts of black tea leaves, Japanese radish leaves, *takana*, and kale were confirmed to have the glycosides converted into aglycones by the xylanase more efficiently than the quinoa extract.

### A3-4. Conversion of Glycosides into Aglycones by Combination of Enzymes

A hot-water extract of horseradish leaves was prepared and adjusted to give 50 ug/mL of KMP aglycones with a pH of 5.0. A xylanase (Hemicellulase Amano 90) was added to the prepared reaction solution to give the final concentrations shown in the table below. Lactase or β-glucosidase (Aromase) was further added to give the final concentrations shown in the table below, and a reaction was allowed to proceed at 50°C for 4 hours, followed by measuring the KMP aglycone concentration by HPLC, thereby confirming the effect of a combination of multiple enzymes. The aglycone formation rate was indicated based on the KMP aglycone concentration during acid hydrolysis taken as 100%.

As shown in Table 2, a combination of xylanase with lactase or β-glucosidase was confirmed to increase the efficiency in converting glycosides into aglycones synergistically.

**Table 2**

| Type of Enzyme | KMP Aglycone Formation Rate (%) | | | |
|---|---|---|---|---|
| | Pre | 1 h | 2 h | 4 h |
| Hemicellulase (250) + Lactase (150) | 0.0 | 41.6 | 67.9 | 89.7 |
| Hemicellulase (250) + Aromase (50) | 0.0 | 38.1 | 61.2 | 90.4 |
| Hemicellulase (250) | 0.0 | 24.7 | 42.6 | 64.8 |
| Hemicellulase (500) | 0.0 | 49.8 | 72.4 | 95.2 |
| Lactase (500) | 0.0 | 7.7 | - | - |
| Aromase (500) | 0.0 | 5.8 | - | - |

### A3-5. Optimum Conditions for Conversion of Glycosides into Aglycones by Xylanase

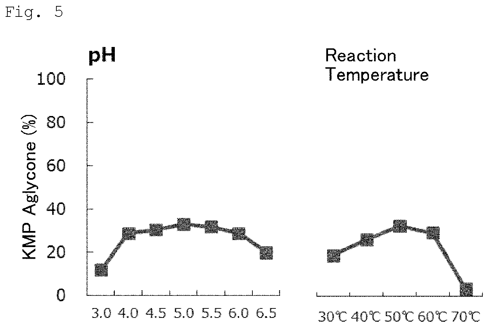

A hot-water extract of horseradish leaves was prepared and suspended to give 50 ug/mL of KMP aglycones. The suspension was adjusted to have a pH of 3 to 6.5, and a xylanase (Hemicellulase Amano 90) was added to give a final concentration of 500 µg/mL. A reaction was allowed to proceed at 50°C for 30 minutes, and pH dependence was investigated. A hot-water extract of horseradish leaves was adjusted to have a pH of 5.0, and a xylanase (Hemicellulase Amano 90) was added to give a final concentration of 500 µg/mL. A reaction was allowed to proceed at 30 to 70°C for 30 minutes, and the KMP aglycone concentration was measured by HPLC, followed by confirming the optimum conditions for enzyme reaction. The aglycone formation rate was indicated based on the KMP aglycone concentration during acid hydrolysis taken as 100%. As shown in Fig. 5, the optimum active pH of the xylanase was 4 to 6, and the optimum active temperature was around 50°C.

### A3-6. Analysis of Conversion of Glycosides into Aglycones

A hot-water extract of horseradish leaves was prepared and adjusted to give 50 ug/mL of KMP aglycones with a pH of 5.0. A xylanase (Hemicellulase Amano 90) was added to the prepared reaction solution to give a final concentration of 1000 µg/mL, and a reaction was allowed to proceed at 50°C for 30, 60, and 120 minutes, followed by observing an HPLC analysis chart to confirm whether a peak of the intermediate of horseradish was present.

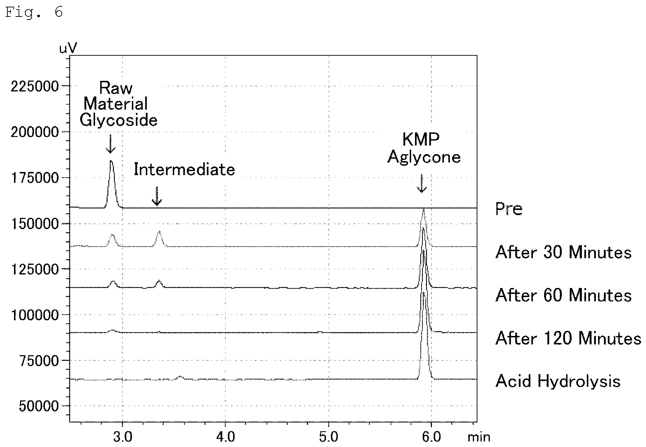

As shown in Fig. 6, the conversion of KMP glycosides derived from horseradish leaves into aglycones by a xylanase was confirmed to take place via an intermediate. From the results of sections 2-1, 2-2, and 2-4 above, it is speculated that in the conversion of KMP glycosides of horseradish leaves into aglycones by a xylanase or β-glucosidase (lactase), an intermediate first forms due to the action of the xylanase, and then the action of β-glucosidase on the intermediate forms KMP aglycones.

### A4. Conversion of Glycosides into Aglycones by Microorganism

A hot-water extract of horseradish leaves was adjusted to give 50 pg/mL, and 188 bacterial strains isolated from food were separately inoculated. After inoculation, aerobic culture was performed at 37°C. After 24 and 120 hours, the KMP aglycone concentration was measured by HPLC to investigate whether microorganism culture converts KMP glycosides of horseradish into KMP aglycones. The results indicate that as shown in Table 3 below, lactic acid bacteria such as *Lactobacillus plantarum*, *L. brevis*, *L. tucceti*, *L. sakei*, *L. pentosus*, *L. casei*, and *L. kefirgranum* isolated from fermentation products such as *mibuna* pickles, *shibazuke* pickles, Chinese cabbage pickles, kimchi, fermented tea, fermented pork, fermented fish, and *sake koji* can efficiently convert KMP glycosides derived from horseradish leaves into aglycones by 95% or higher in 24 hours.

**Table 3**

| Aglycone formation Rate | Fermentation Time | Bacterial Strain |
|---|---|---|
| >95% | 24 hr | *L. pentoses*, *L. plantarum*, *L. brevis*, *L. tucceti* |
| >95% | 120 hr | *L. casei*, *L. sakei* |
| >50% | 120 hr | *L. kefirgranum* |

### A5. Component Analysis

A 50% ethanol extract of horseradish leaves was condensed under reduced pressure to remove ethanol and adjusted to give 3 mg/mL of KMP aglycones with a pH of 5.0. A xylanase (Hemicellulase Amano 90) was added to the prepared reaction solution to give a final concentration of 3 mg/mL, and lactase was added to give a final concentration of 0.9 mg/mL, followed by a reaction at 50°C for 2 hours. Thereafter, the reaction product was treated at 105°C for 15 minutes to inactivate the enzyme, and freeze-dried. Compounds were then extracted from the obtained enzyme-treated horseradish leaf extract sample with methanol. Insoluble and low-polarity components were removed from the extract by using a filter and a spin column. The same extraction operation was also performed for no specimen to make a mock sample. The mock sample was prepared for checking and removing background noise introduced during sample preparation or LC-MS analysis. Each extract was subjected to LC-MS for metabolome analysis.

The results showed that the enzyme treatment significantly increased the content of quercetin and ferulic acid in addition to KMP aglycones, as shown in Table 4 below. Quercetin is known to have physiological action such as antioxidant effects, anti-inflammatory effects, anti-arteriosclerosis effects, cerebrovascular disease prevention, antitumor effects, antihypertensive effects, and vasorelaxant effects. Ferulic acid is known to have physiological action such as antioxidant effects, and antitumor effects.

**Table 4**

| Substance | Measurement value | Note |
|---|---|---|
| KMP | 10 mg | 13.36 million-fold |
| Quercetin | 0.48 mg | 200,000-fold |
| Ferulic acid | 0.09 mg or less | 40-fold |

### B1. Measurement of KMP Glycoside Concentration in Radish Sprout in Low-oxygen Cultivation

### B1-1. Cultivation of Radish Sprout

Radish sprouts were seeded into watered, absorbent cotton such that the seeds did not overlap. The Radish sprouts were grown in an oxygen concentration controlled to 21.0 vol% or 17.5 vol% with a ProOx 110 oxygen controller and a chamber (Kyodo International, Inc.) for 1 week. The oxygen concentration was controlled (decreased) by filling the chamber with nitrogen.

### B1-2. Measurement of Kaempferol and Quercetin

The amount of kaempferol and quercetin was measured after all of the glycosides of kaempferol and quercetin were hydrolyzed and converted into aglycones. The actual procedure was as follows. After each sample was milled, 2.0 g of the milled product was weighed. After 40 mL of 70% ethanol was added, the sample was suspended with a Polytron homogenizer at room temperature at 20,000 rpm for 1 minute. 1 mL of the suspension was dispensed and weighed into a glass test tube, and 1 mL of 2N hydrochloric acid was added. After being mixed with a vortex, the mixture was heated at 100°C for 30 minutes with a heat block. After heating was completed, the resulting product was cooled with ice for at least 5 minutes. After cooling, 5 mL of hexane was added, and the mixture was shaken horizontally 20 times. After centrifugation at 3,000 rpm for 5 minutes at 4°C with a cooled centrifuge, the upper layer was discarded. 5 mL of ethyl acetate was added, and the mixture was shaken horizontally with a shaker at room temperature at 250 rpm for 10 minutes. After centrifugation with a large cooled centrifuge at 4°C at 3,000 rpm for 5 minutes, the upper layer was collected in a glass test tube for drying. 5 mL of ethyl acetate was added again to the lower layer, and the mixture was shaken horizontally with a shaker at room temperature at 250 rpm for 10 minutes. After centrifugation at 3,000 rpm at 4°C for 5 minutes, the upper layer was collected in a glass test tube for drying. The collected upper layer was dried with nitrogen at 60°C with a nitrogen spray dryer. After drying was complete, 1 mL of an aqueous solution of 70% ethanol was added, and the mixture was treated with an ultrasonic generator for 1 minute to dissolve it with a vortex. After being passed through a 0.45-µm filter, the solution was subjected to HPLC to perform KMP aglycone analysis, thereby determining the amount of KMP aglycones.

### B1-3. Results

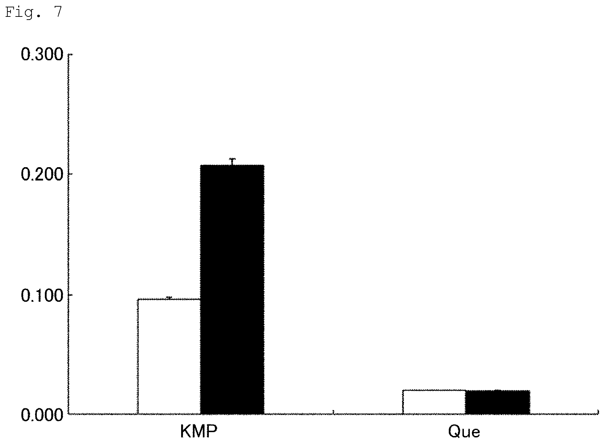

Fig. 7 shows the measured amounts of kaempferol and quercetin in radish sprouts grown in an oxygen concentration of 21 vol% or 17.5 vol%. The KMP content in the radish sprouts grown in an oxygen concentration of 17.5 vol% was 0.207±0.005 mg/g (average ± standard deviation, the same applies below), which was significantly increased about two-fold as compared with the KMP content in the radish sprouts grown according to an ordinary cultivation method in an oxygen concentration of 21 vol% (KMP content: 0.096±0.002 mg/g). However, the quercetin content in the radish sprouts grown in low oxygen and the quercetin content in the radish sprouts grown under ordinary conditions were respectively 0.02±0.00 mg/g and 0.02±0.01 mg/g, indicating no effect of low-oxygen cultivation. These results reveal that decreasing the oxygen concentration during cultivation increases the production of kaempferol.

### B2. Effect of Low-oxygen Cultivation on Phenol Biosynthesis Pathway

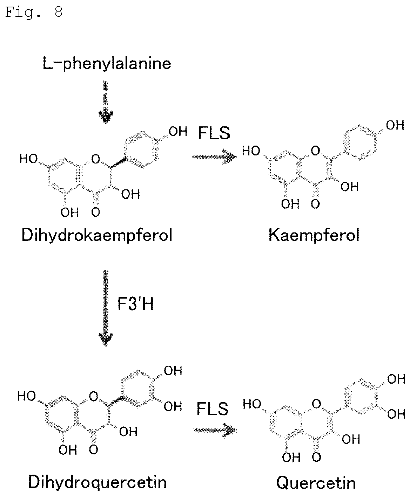

The effect of low-oxygen cultivation on the phenol biosynthesis pathway was studied. Specifically, the effects of low-temperature cultivation on the expression levels of flavonol synthase (FLS) involved in both the synthesis of kaempferol from dihydrokaempferol and the synthesis of quercetin from dihydroquercetin, and on the expression levels of flavonoid 3'-hydroxylase (F3'H) involved in the synthesis of dihydroquercetin from dihydrokaempferol were measured according to the following procedure. Fig. 8 shows the involvement of FLS in the synthesis of kaempferol from dihydrokaempferol and in the synthesis of quercetin from dihydroquercetin, and the involvement of F3'H in the synthesis of dihydroquercetin from dihydrokaempferol.

### B2-1. Cultivation Method for Radish Sprouts and Broccoli

Radish sprouts and broccoli were seeded into watered, absorbent cotton such that the seeds did not overlap. The radish sprouts and broccoli were grown in an oxygen concentration controlled to 21.0 vol% or 17.5 vol% with a ProOx 110 oxygen controller and a chamber (Kyodo International, Inc.) for 1 week. The oxygen concentration was controlled (decreased) by filling the chamber with nitrogen.

### B2-2. Measurement of Gene Expression Level

The samples (all plants) were individually frozen in liquid nitrogen and crushed with a mortar and a pestle, followed by weighing 100 mg of the ground product. Total RNA was extracted using an RNeasy Plant Mini Kit (Qiagen, Valencia, CA), and reverse-transcribed to cDNA by using a High-Capacity cDNA Reverse Transcription Kit (Thermo Fisher Scientific, Inc., Waltham, MA). PCR was performed by using TaqMan Gene Expression Assays (flavonoid 3'-hydroxylase (F3'H), flavonol synthase (FLS), actin) and a TaqMan Fast Advanced Master Mix (Thermo Fisher Scientific, Inc.) with a QuantStudio^{™} 3 Real-Time PCR System (Thermo Fisher Scientific, Inc.). Actin was used as an endogenous control for quantification.

### B2-3. Results

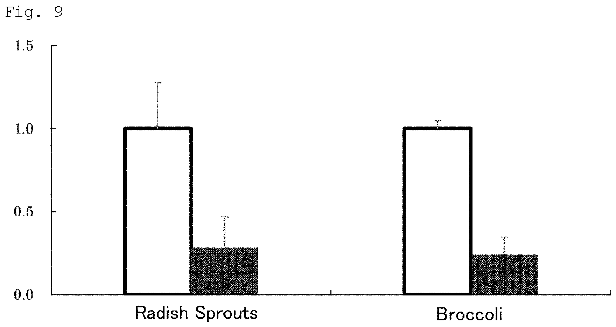

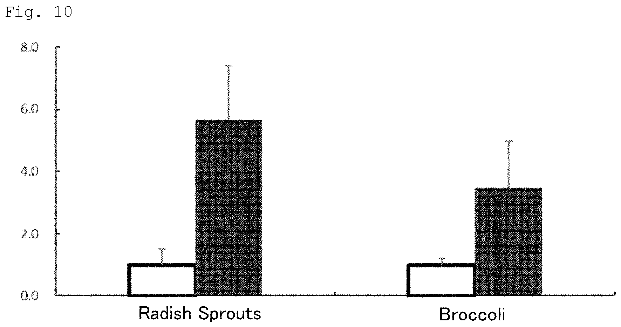

Fig. 9 shows the results of measuring the expression levels of the F3'H gene. Fig. 10 shows the results of measuring the expression levels of the FLS gene. The results indicate that when grown in an oxygen concentration of 17.5 vol% in which the kaempferol content was confirmed to increase, radish sprouts and broccoli both exhibited significantly decreased expression levels of the F3'H gene compared with the expression levels of the F3'H gene in an ordinary cultivation method (oxygen concentration 21: vol%) taken as 1. The expression levels of FLS were also found to have significantly increased in both radish sprouts and broccoli when they were grown in an oxygen concentration of 17.5 vol% compared with when grown in an oxygen concentration of 21 vol%. These results suggest that cultivation under low-oxygen conditions activates metabolic reactions from dihydrokaempferol to kaempferol and inhibits the synthesis of other substances from dihydrokaempferol in plants producing kaempferol, such as cruciferous plants.

### B3. Effect of Low-oxygen Cultivation on KMP Production in Horseradish

### B3-1. Cultivation of Horseradish

Rhizomes of horseradish were planted in soil and grown in an oxygen concentration controlled with a ProOx 110 oxygen controller and a chamber (Kyodo International, Inc.) to 21.0 vol%, 18.5 vol%, 17.5 vol%, or 16.5 vol% for 1 week. The oxygen concentration was controlled (decreased) by filling the chamber with nitrogen.

### B3-2. Method for Measuring Length of Leaf and Petiole

Leaves and petioles were cut from each sample and measured with a ruler.

### B3-3. Results

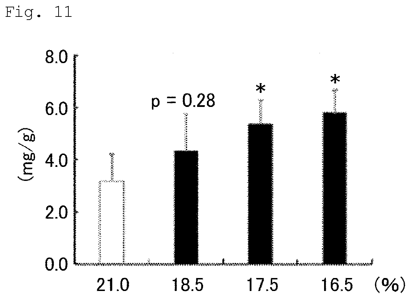

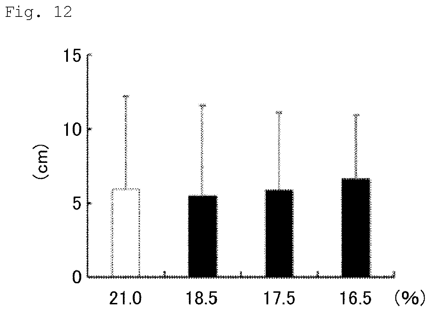

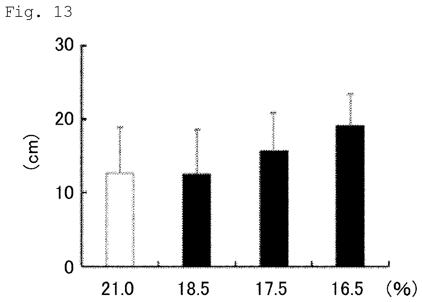

Fig. 11 shows the amount of kaempferol measured in the same manner as in section 1-2 above for the horseradish leaves grown in different oxygen concentrations. The results indicate that the amount of produced kaempferol increased with a decrease in the oxygen concentration during cultivation. As shown in Figs. 12 and 13, there was no significant effect of limited oxygen concentrations on the length of leaves and stalks.

### C. Enhancement of KMP Synthesis by Addition of Phenylalanine and Tyrosine

### C-1. Biosynthesis Pathway from Phenylalanine or Tyrosine into KMP

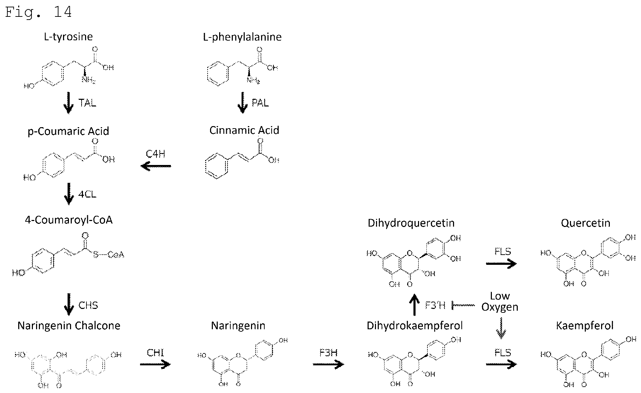

L-tyrosine and L-phenylalanine serve as indirect raw materials in kaempferol biosynthesis. As shown in Fig. 14, L-tyrosine can be converted to kaempferol via p-coumaric acid, 4-coumaroyl-CoA, naringenin chalcone, naringenin, and dihydrokaempferol. L-phenylalanine can be converted to kaempferol via cinnamic acid, p-coumaric acid, 4-coumaroyl-CoA, naringenin chalcone, naringenin, and dihydrokaempferol.

### C-2. L-tyrosine or L-phenylalanine Addition Effect 1

Aqueous solutions containing L-phenylalanine in a concentration of 25, 50, 100, 200, or 400 mg/L, aqueous solutions containing L-tyrosine in a concentration of 25, 50, 100, 200, or 400 mg/L, and aqueous solutions containing both L-tyrosine and L-phenylalanine in a concentration of 25, 50, 100, 200, or 400 mg/L were prepared. As test plants, radish sprouts and broccoli sprouts were prepared. The test plants were given one of the aqueous solutions in an amount of 10 ml/day for 7 days and grown in the dark in an oxygen concentration of 21 vol%. After 7 days, the content of kaempferol and quercetin was measured for each test plant in the same manner as in section B1-2 above. Fig. 15 shows the measurement results of radish sprouts and Fig. 16 shows the measurement results of broccoli sprouts. The results indicate that adding L-tyrosine and/or L-phenylalanine increased the kaempferol content in both the radish sprouts and broccoli sprouts. There was no significant effect of L-tyrosine and/or L-phenylalanine on the quercetin content in radish sprouts. Additionally, quercetin was not detected in broccoli sprouts regardless of the addition of L-tyrosine and L-phenylalanine. There was also no clear effect of the addition of L-tyrosine and/or L-phenylalanine on plant growth (total length and weight).

### C-3. L-tyrosine or L-phenylalanine Addition Effect 2

Aqueous solutions containing L-phenylalanine in a concentration of 25 or 50 mg/L, and aqueous solutions containing L-tyrosine in a concentration of 25 or 50 mg/L were prepared. As a test plant, radish sprouts were prepared. The test plant was given one of the aqueous solution in an amount of 10 ml/day for 7 days and grown in the dark in an oxygen concentration of 21 vol% or 17.5 vol%. After 7 days, the content of kaempferol and quercetin was measured in the same manner as in section Bl-2 above. As shown in Fig. 17, the results indicate that adding L-tyrosine or L-phenylalanine increased the kaempferol content in every oxygen concentration, and that adding L-tyrosine or L-phenylalanine significantly increased the rate of increase in the kaempferol content in a low-oxygen concentration (17.5 vol%). This indicates that the addition of L-tyrosine or L-phenylalanine is more effective in cultivation in a low-oxygen concentration. There was no clear effect of the addition of L-tyrosine or L-phenylalanine on plant growth (total length and weight).

## Claims

1. A plant extract comprising a kaempferol aglycone in an amount of 1 mg/g or more on a dry-weight basis.

2. The plant extract according to claim 1, comprising the kaempferol aglycone in an amount of 100 mg/g or more.

3. The plant extract according to claim 1 or 2, further comprising quercetin in an amount of 0.1 mg/g or more on a dry-weight basis.

4. The plant extract according to any one of claims 1 to 3, which is an extract of a horseradish leaf.

5. A method for producing the plant extract of any one of claims 1 to 4, the method comprising
(1) subjecting a plant material containing a kaempferol glycoside to extraction with a solvent, and
(2) subjecting the extract obtained in step (1) to hydrolysis treatment.

6. The method according to claim 5, wherein the hydrolysis treatment comprises allowing an enzyme or a microorganism to act on the extract.

7. The method according to claim 5 or 6, wherein the hydrolysis treatment comprises allowing an enzyme to act on the extract, and the enzyme contains a xylanase.

8. A method for producing a plant with an increased content of kaempferol and/or a kaempferol glycoside per unit weight, the method comprising growing a plant under the following condition:
an oxygen concentration of 19 vol% or lower, or
a partial pressure of oxygen of 193 hPa or lower.

9. A method for increasing a kaempferol and/or kaempferol glycoside production ability of a plant, the method comprising growing a plant under the following condition:
an oxygen concentration of 19 vol% or lower, or
a partial pressure of oxygen of 193 hPa or lower.

10. A method for producing a plant with an increased kaempferol and/or kaempferol glycoside production ability, the method comprising growing a plant under the following condition:
an oxygen concentration of 19 vol% or lower, or
a partial pressure of oxygen of 193 hPa or lower.

11. The method according to any one of claims 8 to 10, wherein the plant is a cruciferous plant.

12. A plant obtained by the method of any one of claims 8 to 11.

13. A method for producing an extract containing kaempferol and a kaempferol glycoside, the method comprising extracting the kaempferol and kaempferol glycoside from the plant of claim 12.
